# EUROPÄISCHE PATENTANMELDUNG

(11) **EP 1 306 660 A2**
(43) Veröffentlichungstag der Anmeldung: **02.05.2003**
(21) Anmeldenummer: 02023465.4
(22) Anmeldetag: 21.10.2002
(51) Int. Cl.: G01N 11/00, B25J 9/00, B25J 15/00, G01N 35/00, G01N 3/00, G01N 33/44

(54) **Vorrichtung und Verfahren zum Messen/Prüfen physikalischer Eigenschaften an Materialproben**

(30) Priorität: 25.10.2001 DE 10152041
(71) Anmelder: GÖTTFERT WERKSTOFF-PRÜFMASCHINEN GMBH, D-74722 Buchen (DE)
(72) Erfinder: Göttfert, Axel, Dr.-Ing., 74722 Buchen (DE)
(74) Vertreter: Naumann, Ulrich, Dr.-Ing.

(57) **Zusammenfassung**

Vorrichtung und Verfahren zum Messen/Prüfen physikalischer Eigenschaften an Materialproben mit einer eine Prüfform (2) aufweisenden Prüfkammer (1) und einer Fördereinrichtung (3) zum Verbringen der Materialproben (10) von einer Bereitstellungsposition in die Prüfposition, wobei die Materialproben (10) in der Bereitstellungsposition zur Verhinderung eines unmittelbaren Kontakts mit der Prüfform (2) verpackt, umhüllt oder beidseitig abgedeckt werden, wobei die Fördereinrichtung (3) eine Aufnahme (5) für die verpackte Materialprobe (10) umfasst, die gemeinsam mit der Materialprobe (10) von der Bereitstellungsposition in die Prüfposition und gegebenenfalls in eine Abgabeposition schwenkbar ist bzw geschwenkt werden kann.

## Beschreibung

Die Erfindung betrifft eine Vorrichtung sowie ein Verfahren zum Messen/Prüfen physikalischer Eigenschaften an Materialproben mit einer eine Prüfform aufweisenden Prüfkammer und einer Fördereinrichtung zum Verbringen der Materialproben von einer Bereitstellungsposition in die Prüfposition, wobei die Materialproben in der Bereitstellungsposition zur Verhinderung eines unmittelbaren Kontakts mit der Prüfform verpackt, umhüllt oder beidseitig abgedeckt werden.

Bisher sind Vorrichtungen und Verfahren zum Messen/Prüfen physikalischer Eigenschaften an Materialproben bekannt, bei denen die Materialproben in einer Bereitstellungsposition von einer Kunststofffolie beidseits abgedeckt werden. Dies dient vor allem einer Vermeidung eines unmittelbaren Kontakts der Materialprobe mit den Prüfmitteln, um Verschmutzungen oder Beschädigungen der Prüfmittel zu vermeiden.

Aus dem europäischen Patent 0 511 189 ist eine Vorrichtung und ein Verfahren bekannt, bei dem eine Materialprobe zwischen zwei Endlosfolien, die von zwei Rollen abgerollt werden, in eine Prüfkammer transportiert wird. Nach Beendigung der Messung wird die Materialprobe, welche sich aufgrund von Temperatur- und Druckeinwirkung fest in die Folie eingedrückt hat, aus der Prüfkammer gezogen. Hierbei haben die Endlosfolien die Funktion eines Förderbandes, das die Materialprobe sowohl in die Prüfkammer als auch von der Prüfkammer wegführt.

Ein entscheidender Nachteil dieser Vorrichtung ist die Tatsache, dass in der Kunststofffolie eine gewisse Vorspannung aufrechterhalten wird, die darin begründet ist, dass die Kunststofffolie als Transportband zum Ziehen der Materialprobe ausgelegt ist. Diese Vorspannung kann die Messung stören und die Messergebnisse verfälschen.

Nachteilig ist außerdem, dass bei der Entnahme der Materialprobe aus der Prüfkammer die Kunststofffolie über eine Fläche der Prüfform herausgezogen wird, da bei diesem Vorgang Scherkräfte in horizontaler Richtung auftreten, die zum unkontrollierten Reißen der Kunststofffolie und somit zu einer Unterbrechung des gesamten Arbeitsablaufs führen können. Um ein Reißen der Kunststofffolie zu vermeiden, müssen daher relativ dicke und stabile Folien verwendet werden, deren Dicke und Materialeigenschaften wiederum die Messung störend beeinflussen können.

Um eine störungsfreie Messung durchzuführen, muss bei Verwendung einer dünnen Kunststofffolie die Geschwindigkeit des Transportbandes soweit verringert werden, dass deren Reißen ausgeschlossen ist. Dies wirkt sich dann vor allem nachteilig auf die Taktzeit eines Messvorgangs aus.

Der vorliegenden Erfindung liegt nunmehr die Aufgabe zugrunde, eine Vorrichtung sowie ein Verfahren zum Messen/Prüfen physikalischer Eigenschaften an Materialproben der eingangs genannten Art anzugeben, wonach einerseits eine Minimierung der Störeinflüsse der Verpackung beim Messen und andererseits ein problemloses Zuführen und Entnehmen der Materialproben aus der Prüfkammer erreicht sind.

Erfindungsgemäß wird die voranstehende Aufgabe hinsichtlich einer Vorrichtung zum Messen/Prüfen physikalischer Eigenschaften an Materialproben durch eine Vorrichtung mit den Merkmalen des Patentanspruchs 1 gelöst. Danach ist eine Vorrichtung zum Messen/Prüfen physikalischer Eigenschaften an Materialproben der eingangs genannten Art derart ausgestaltet, dass die Fördereinrichtung eine Aufnahme für die verpackte Materialprobe umfasst, die gemeinsam mit der Materialprobe von der Bereitstellungsposition in die Prüfposition und ggf. in eine Abgabeposition schwenkbar ist.

Des Weiteren ist die obige Aufgabe im Hinblick auf ein Verfahren zum Messen/Prüfen physikalischer Eigenschaften an Materialproben durch ein Verfahren mit den Merkmalen des Patentanspruchs 22 gelöst. Danach ist ein Verfahren zum Messen/Prüfen physikalischer Eigenschaften an Materialproben der eingangs genannten Art derart ausgestaltet, dass die Fördereinrichtung eine Aufnahme für die verpackte Materialprobe umfasst, die gemeinsam mit der Materialprobe von der Bereitstellungsposition in die Prüfposition und ggf. in eine Abgabeposition geschwenkt wird.

In erfindungsgemäßer Weise ist erkannt worden, dass durch die Vorkehrung einer Fördereinrichtung, die eine schwenkbare Aufnahme umfasst, die Nachteile einer als Transportmittel dienenden Verpackung wirksam vermieden werden können. Die Verpackung der Materialproben unterliegt bei der erfindungsgemäßen Ausgestaltung keiner Vorspannung durch Zugkräfte, die auf ein Transportband wirken. Insofern ist eine Störung der Messung durch eine vorgespannte Verpackung ausgeschlossen. Der Schwenkvorgang erfordert außerdem keine besonders dicke und stabile Verpackung, da eventuelle Beschädigungen der Verpackung durch den Transport der Materialprobe insofern unkritisch sind, als sie den Transport weiterer Materialproben und somit den Arbeitsablauf nicht stören können. Die erfindungsgemäße Ausgestaltung erlaubt daher die Verwendung einer für die Messung geeigneten, dünnen Verpackung, die keinen störenden Einfluss auf die Messergebnisse hat.

Genauer gesagt wird durch das Verschwenken der Materialprobe die Funktion der Verpackung als Verpackungsmittel von ihrer Funktion als Transportmedium entkoppelt. Die erfindungsgemäße Ausgestaltung erlaubt durch diese Entkopplung nicht nur die Verwendung einer dem Messprozess optimal angepassten Verpackung, sondern auch eine von der Beschaffenheit der Verpackung nahezu unabhängige Wahl der Geschwindigkeit eines Messtakts. Insofern können Materialproben der Prüfkammer problemlos zugeführt, weitgehend störungsfrei gemessen und wieder entnommen werden.

In besonders vorteilhafter Weise könnte die Fördereinrichtung einen Schwenkarm umfassen, an dem die Aufnahme angeordnet ist. Dadurch wäre gewährleistet, dass die Aufnahme einen geeigneten Abstand von der Schwenkachse einnimmt und daher die Materialprobe beim Verschwenken nicht mit der Schwenkachse in Kontakt kommt. Verwicklungen der Verpackung mit der Achse sind insofern ausgeschlossen.

Ein besonders komfortables Ergreifen der verpackten Materialprobe könnte durch eine als Zange ausgebildete Aufnahme erreicht werden. Dadurch wäre außerdem gewährleistet, dass die Materialprobe besonders fest und nur an definierten Stellen gegriffen werden kann.

In besonders vorteilhafter Ausgestaltung könnte die Zange zweigeteilt sein, wodurch ein komfortables Greifen der Materialprobe von zwei Seiten ermöglicht wäre.

Dadurch wäre außerdem realisierbar, dass die Materialprobe besonders schonend in symmetrischer Weise gleichzeitig erfasst würde.

Eine besonders verpackungsschonende Entfernung der Materialprobe aus der Prüfform könnte dadurch erzielt werden, dass die Zange vertikal heb- und/oder senkbar ist. Durch eine solche Ausgestaltung könnten Scherkräfte beim Entformen der Materialprobe völlig vermieden werden, da das Heben oder Senken lediglich Zugkräfte auf die Verpackung ausüben würde.

In besonders vorteilhafter Weise könnten die Greifflächen der geöffneten Zange einen Winkel von 180 Grad einschließen. Dadurch wäre gewährleistet, dass die Zange beim Schwenkprozess mit geöffneten Greifflächen direkt vor oder neben die Materialprobe gebracht werden kann, ohne diese mit den Greifflächen zu berühren. Die Materialprobe kann dann durch Schließen der Zange von zwei Seiten ergriffen werden.

In einer ganz besonders vorteilhaften Ausgestaltung könnten die Greifflächen der Zange zwei gegenüberliegende Ringe mit je einem Ringspalt an der gleichen Stelle sein. Dadurch wäre das Greifen einer verpackten Materialprobe ermöglicht, die auf einer beispielsweise runden Plattform liegt, die lediglich mit einem Steg an der Vorrichtung angeordnet ist. Die Greifflächen würden dabei um die Plattform herumgreifen, ohne diese oder deren Steg zu berühren, lediglich die Verpackung würde durch die Zange erfasst. Dadurch wäre eine Entnahme der Materialprobe von der Plattform in der Weise möglich, dass die Greifflächen der Zange durch die Plattform hindurch schwenken. Die Ringe könnten dabei den vier- bis fünffachen Durchmesser der Materialprobe haben, so dass die Probe nicht direkt mit der Zange in Kontakt käme. Insofern wäre ein besonders schonender Transport der Materialprobe realisiert.

In besonders einfacher Weise könnte die Materialprobe zwischen zwei Lagen einer Verpackung gebracht werden, wenn eine Lage auf eine Plattform schiebbar, die Materialprobe auf der Lage positionierbar, die Plattform nach der Positionierung der Materialprobe absenkbar und eine weitere Lage über die Materialprobe schiebbar wäre. Insofern wäre realisiert, dass die Materialprobe definiert verpackt würde.

Eine Positionierung der Materialprobe auf der Plattform könnte mit einem pneumatischen Greifarm erfolgen. Dadurch wäre realisiert, dass der Verpackungsprozess völlig automatisch abläuft. Die Positionierung durch einen pneumatischen Greifarm würde außerdem ebenfalls eine definierte Positionierung der Materialprobe auf der Lage bzw. Plattform zur Folge haben.

Hinsichtlich einer besonders exakten Anordnung der Lagen auf der Plattform könnten diese über eine Zuführeinrichtung auf die Plattform führbar sein, hierdurch wäre eine definierte und vor allem reproduzierbare Positionierung der Lagen ermöglicht.

Ein exaktes Positionieren der Materialprobe ohne Verrutschen der Lage könnte erzielt werden, wenn die Lagen mit einer Befestigungsvorrichtung auf der Plattform fixierbar wären.

Hinsichtlich einer besonders schnellen Zuführung der Lagen auf die Plattform könnten diese auf einer Rolle aufgewickelt sein, von der sie abgewickelt direkt auf die Plattform geführt werden.

Für eine Abtrennung der Lagen von der Rolle oder deren Zuschneiden auf eine bestimmte Länge könnte in vorteilhafter Weise ein Laufmesser vorgesehen sein, das die Lagen von der Rolle abtrennt. Die Lagen könnten dabei in einer Länge von 10 cm abgetrennt werden. Durch das Abtrennen mit einem Laufmesser wird auf sogenannte Sollbruchstellen bei einem Endlosmaterial verzichtet, so dass insofern eine erhebliche Kosteneinsparung bei der Wahl des Verpackungsmaterials erzielt werden kann.

Hinsichtlich einer besonders leichten Zugänglichkeit der verpackten Materialprobe von allen Seiten könnte die Plattform eine runde Scheibe sein, die nur mit einem Steg an der Vorrichtung befestigt ist.

In besonders vorteilhafter Weise könnte die Zange so ausgebildet sein, dass die Greifflächen der Zange zwei gegenüberliegende Ringe mit je einem Ringspalt an der gleichen Stelle sind. Dadurch wäre ermöglicht, dass die Zange in der Bereitstellungsposition so um die Plattform bringbar ist, dass die Ringe konzentrisch um die Plattform liegen und der Steg im Ringspalt steht. Durch eine solche Aufnahme der verpackten Probe wird ermöglicht, dass die Greifflächen von oben und unten kommend die Materialprobe zwischen zwei Lagen durch Einklemmen derselben mit den Greifflächen einschließen kann. Hinsichtlich einer besonders symmetrischen Belastung der Materialprobe und des Verpackungsmaterials könnte die Zange die Materialprobe mittig aufnehmen.

Die Zange könnte in der Prüfposition so um die Prüfform bringbar sein, dass die Ringe der Zange konzentrisch um eine runde Prüfform liegen und die Materialprobe zwischen den Lagen in der Prüfform positioniert ist. Hierdurch wäre in besonders vorteilhafter Weise realisiert, dass die Zange während der Messung in der Prüfposition verbleiben kann, beispielsweise wenn auf die Prüfform von oben eine weitere runde Prüfform herabfährt. Dabei wäre die Materialprobe mittig in der Prüfform eingeschlossen und die Zange würde konzentrisch um die runde Prüfform liegen. Eine solche Prüfkammer könnte eine Prüfkammer nach ISO 6502 sein.

Um den Störeinfluss während der Messung zu minimieren könnten die Lagen der Verpackung weniger als 0,5 mm dick sein. Hinsichtlich eines besonders kostengünstigen Messvorgangs könnten die Lagen Kunststofffolien sein.

Um den Messvorgang komplett zu automatisieren, könnte die Materialprobe aus einem Materialprobenmagazin, vorzugsweise über einen mechanischen oder elektrischen Code, selektierbar sein. Dadurch wäre ein eindeutiges Identifizieren von Materialproben gewährleistet.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche andererseits auf die nachfolgende Erläuterung eines bevorzugten Ausführungsbeispiels der erfindungsgemäßen Vorrichtung anhand der Zeichnung zu verweisen. In Verbindung mit der Erläuterung des bevorzugten Ausführungsbeispiels anhand der Zeichnung werden auch im allgemeinen bevorzugte Ausgestaltungen und Weiterbildungen der Lehre erläutert. In der Zeichnung zeigt
- Fig. 1: in einer Draufsicht, teilweise und schematisch, ein Ausführungsbeispiel der erfindungsgemäßen Vorrichtung zum Messen/Prüfen physikalischer Eigenschaften, wobei die Greifflächen der Zange um 180 Grad geöffnet sind und die Fördereinrichtung in der Bereitstellungsposition steht;
- Fig. 2: in einer Draufsicht die Vorrichtung aus Fig. 1, wobei eine Lage Verpackungsmaterial auf die Plattform geführt ist;
- Fig. 3: in einer Draufsicht die Vorrichtung aus Fig. 1, wobei die Zange geschlossen ist und die Lagen zwischen ihren Greifflächen einschließt;
- Fig. 4: in einer Draufsicht die Vorrichtung aus Fig. 1, wobei die Fördereinrichtung in die Prüfposition verschwenkt ist und
- Fig. 5: in einer Draufsicht die Vorrichtung aus Fig. 1, wobei die Fördereinrichtung in der Abgabeposition steht.

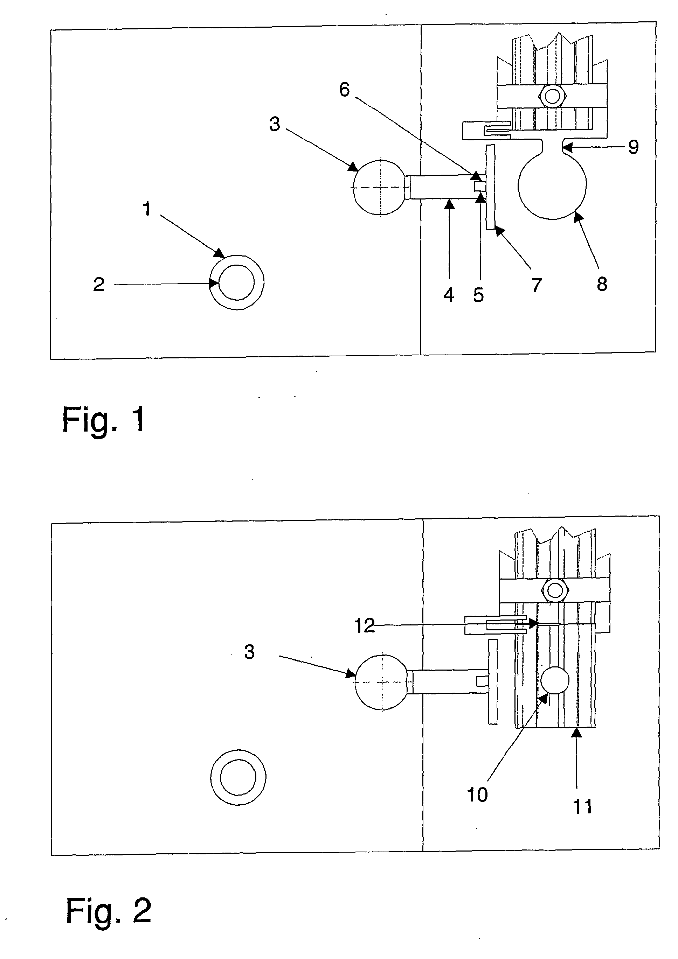

Fig. 1 zeigt ein Ausführungsbeispiel der erfindungsgemäßen Vorrichtung zum Messen/Prüfen physikalischer Eigenschaften an Materialproben in einer schematischen und teilweisen Draufsicht. Die Vorrichtung weist eine Prüfkammer 1, mit einer Prüfform 2 auf. Die Vorrichtung umfasst eine Fördereinrichtung 3 zum Verbringen der Materialproben von einer Bereitstellungsposition in die Prüfposition. Die Fördereinrichtung 3 umfasst einen Schwenkarm 4, an dem die Aufnahme 5 angeordnet ist. Die Aufnahme 5 ist als Zange 6 ausgebildet. Die Zange weist Greifflächen 7 auf, die im geöffneten Zustand einen Winkel von 180 Grad einschließen. Außerdem umfasst die Vorrichtung eine runde Plattform 8, die über einen Steg 9 an der Vorrichtung angeordnet ist.

Fig. 2 zeigt die Zange 6 in geöffnetem Zustand, wobei die Greifflächen 7 einen Winkel von 180 Grad einschließen. Die Materialprobe 10 befindet sich auf einer ersten Lage 11, die auf der Plattform 8 aufliegt. Ein Laufmesser 12 trennt die erste Lage 11 von einer weiteren Lage ab. Die Fördereinrichtung 3 befindet sich in der Bereitstellungsposition.

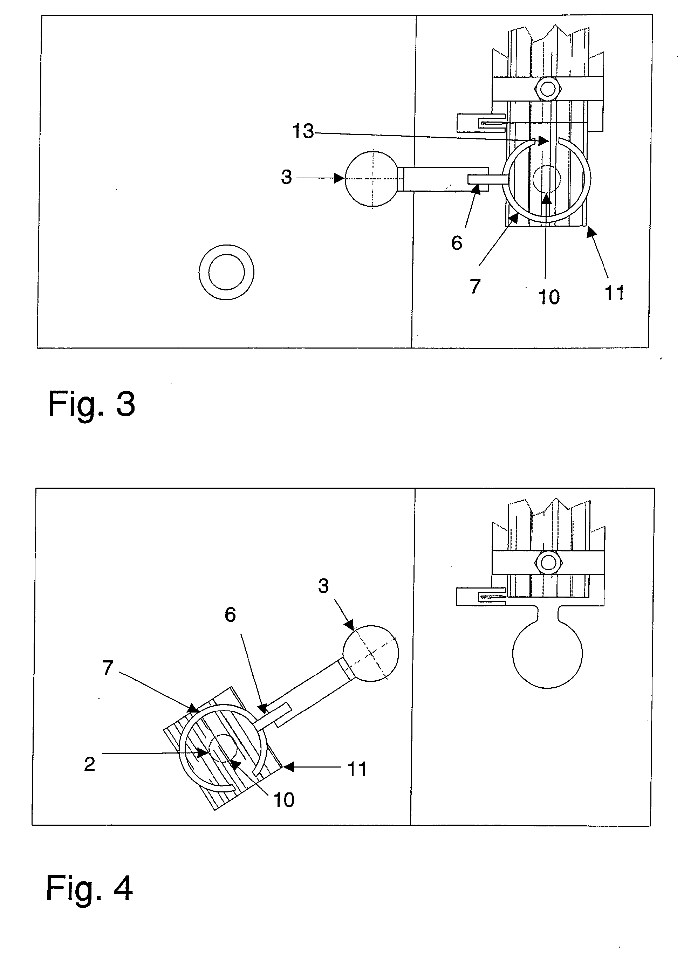

Fig. 3 zeigt die Zange 6 in geschlossenem Zustand. Die Greifflächen 7 der Zange 6 sind zwei gegenüberliegende Ringe mit je einem Ringspalt 13 an der gleichen Stelle. Die Zange 6 schließt mit ihren Greifflächen 7 die Materialprobe 10 zwischen zwei Lagen 11 ein. Die Lagen 11 und die Probe 10 befinden sich auf der abgesenkten Plattform 8. Die Zange 6 ist so um die Plattform 8 angeordnet, dass die Greifflächen 7 konzentrisch um die Plattform 8 liegen und der Steg 9 im Ringspalt 13 steht. Die Zange 6 drückt zwei Lagen 11 mit ihren Greifflächen 7 so zusammen, dass die Materialprobe 10 zwischen den Lagen 11 lose gehalten wird. Die Fördereinrichtung 3 befindet sich in der Bereitstellungsposition.

Fig. 4 zeigt die Fördereinrichtung 3 nach Verschwenken in die Prüfposition. Die Zange 6 ist so um die Prüfform 2 gebracht, dass die Greifflächen 7 konzentrisch um die Prüfform 2 liegen und die Materialprobe 10 zwischen den Lagen 11 in der Prüfform 2 positioniert ist.

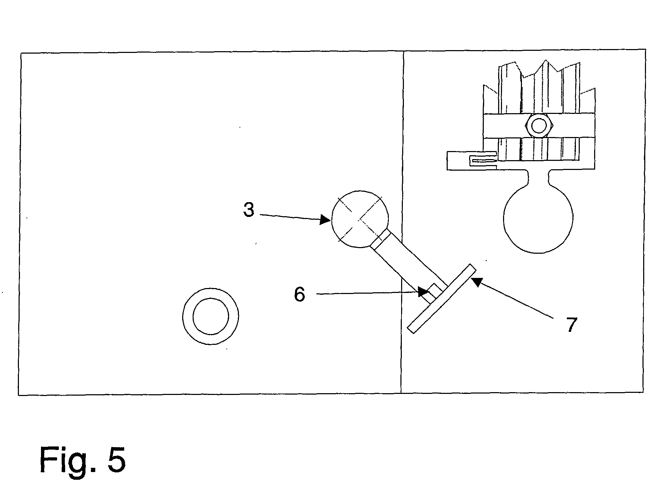

Fig. 5 zeigt die Fördereinrichtung 3 nach Verschwenken in die Abgabeposition. Die Greifflächen 7 der Zange 6 sind um 180 Grad geöffnet, die Materialprobe 10 ist entfernt.

Schließlich sei ausdrücklich darauf hingewiesen, dass das voranstehend beschriebene Ausführungsbeispiel lediglich zur Erörterung der beanspruchten Lehre dient, diese jedoch nicht auf das Ausführungsbeispiel einschränkt.

## Patentansprüche

1. Vorrichtung zum Messen/Prüfen physikalischer Eigenschaften an Materialproben mit einer eine Prüfform (2) aufweisenden Prüfkammer (1) und einer Fördereinrichtung (3) zum Verbringen der Materialproben (10) von einer Bereitstellungsposition in die Prüfposition, wobei die Materialproben (10) in der Bereitstellungsposition zur Verhinderung eines unmittelbaren Kontakts mit der Prüfform (2) verpackt, umhüllt oder beidseitig abgedeckt werden,
**dadurch gekennzeichnet, dass** die Fördereinrichtung (3) eine Aufnahme (5) für die verpackte Materialprobe (10) umfasst, die gemeinsam mit der Materialprobe (10) von der Bereitstellungsposition in die Prüfposition und gegebenenfalls in eine Abgabeposition schwenkbar ist.

2. Vorrichtung nach Anspruch 1, **dadurch gekennzeichnet, dass** die Fördereinrichtung (3) einen Schwenkarm (4) umfasst, an dem die Aufnahme (5) angeordnet ist.

3. Vorrichtung nach Anspruch 1 oder 2, **dadurch gekennzeichnet, dass** die Aufnahme (5) als Zange (6) ausgebildet ist, wobei die Zange (6) vorzugsweise zweigeteilt ist und vertikal heb- und/oder senkbar sein kann.

4. Vorrichtung nach einem der Ansprüche 1 bis 3, **dadurch gekennzeichnet, dass** die Greifflächen (7) der Zange (6) im geöffneten Zustand einen Winkel von 180 Grad einschließen und/oder zwei gegenüberliegende Ringe mit je einem Ringspalt (13) an der gleichen Stelle sind.

5. Vorrichtung nach einem der Ansprüche 1 bis 4, **dadurch gekennzeichnet, dass** in der Bereitstellungsposition eine Lage (11) auf eine Plattform (8) schiebbar, die Materialprobe (10) auf der Lage (11) positionierbar, die Plattform (8) nach Positionieren der Materialprobe (10) absenkbar und eine weitere Lage (11) über die Materialprobe (10) schiebbar ist, wobei die Materialprobe (10) mit einem pneumatischen Greifarm auf der Lage (11) bzw. der Plattform (8) positionierbar sein kann.

6. Vorrichtung nach Anspruch 5, **dadurch gekennzeichnet, dass** die Lagen (11) über eine Zuführeinrichtung auf die Plattform (8) führbar sind und/oder mit einer Befestigungsvorrichtung auf der Plattform (8) fixierbar sind und/oder von einer Rolle, auf der sie aufgewickelt sind, auf die Plattform (8) führbar sind und/oder mit einem Laufmesser (12) von der Rolle abtrennbar sind.

7. Vorrichtung nach einem der Ansprüche 4 bis 6, **dadurch gekennzeichnet, dass** die Plattform (8) eine runde Scheibe ist, die mit einem Steg (9) an der Vorrichtung befestigt ist, wobei die Zange (6) vorzugsweise so um die Plattform (8) bringbar ist, dass die Greifflächen (7) konzentrisch um die Plattform (8) liegen und der Steg (9) im Ringspalt (13) steht.

8. Vorrichtung nach einem der Ansprüche 4 bis 7, **dadurch gekennzeichnet, dass** die Zange (6) die zwei Lagen (11) mit ihren Greifflächen (7) so zusammendrückt, dass die Materialprobe (10) zwischen den Lagen (11) lose gehalten wird, wobei die Zange (6) die Materialprobe (10) mittig aufnehmen kann und/oder wobei die Zange (6) in der Prüfposition so um die Prüfform (2) bringbar ist, dass die Greifflächen (7) konzentrisch um die Prüfform (2) liegen und die Materialprobe (10) zwischen den Lagen (11) in der Prüfform (2) positioniert ist.

9. Vorrichtung nach einem der Ansprüche 1 bis 8, **dadurch gekennzeichnet, dass** die Materialprobe (10) aus einem Materialprobenmagazin, vorzugsweise über einen mechanischen oder elektrischen Code, selektierbar ist.

10. Verfahren zum Messen/Prüfen physikalischer Eigenschaften an Materialproben (10), insbesondere unter Verwendung einer Vorrichtung nach einem der Ansprüche 1 bis 9, mit einer eine Prüfform (2) aufweisenden Prüfkammer (2) und einer Fördereinrichtung (3) zum Verbringen der Materialproben (10) von einer Bereitstellungsposition in die Prüfposition, wobei die Materialproben (10) in der Bereitstellungsposition zur Verhinderung eines unmittelbaren Kontakts mit der Prüfform (2) verpackt, umhüllt oder beidseitig abgedeckt werden,
**dadurch gekennzeichnet, dass** die Fördereinrichtung (3) eine Aufnahme (5) für die verpackte Materialprobe (10) umfasst, die gemeinsam mit der Materialprobe (10) von der Bereitstellungsposition in die Prüfposition und gegebenenfalls in eine Abgabeposition geschwenkt wird.

11. Verfahren nach Anspruch 10, **dadurch gekennzeichnet, dass** in der Bereitstellungsposition eine Lage (11) auf eine Plattform (8) geschoben, die Materialprobe (10) auf der Lage (11) positioniert, die Plattform (8) nach Positionieren der Materialprobe (10) abgesenkt und eine weitere Lage (11) über die Materialprobe (10) geschoben wird, wobei die Materialprobe (10) mit einem pneumatischen Greifarm auf der Lage (11) bzw. der Plattform (8) positioniert wird, die Lagen (11) über eine Zuführeinrichtung ggf. von einer Rolle, auf der sie aufgewickelt sind, auf die Plattform (8) geführt und ggf. mit einer Befestigungsvorrichtung auf der Plattform (8) fixiert und ggf. mit einem Laufmesser (12) von der Rolle abgetrennt werden.

12. Verfahren nach Anspruch 10 oder 11, **dadurch gekennzeichnet, dass** die Zange (6) so um die Plattform (8) gebracht wird, dass die Greifflächen (7) konzentrisch um die Plattform (8) liegen und der Steg (9) im Ringspalt (13) steht.

13. Verfahren nach einem der Ansprüche 10 bis 12, **dadurch gekennzeichnet, dass** die zwei Lagen (11) mit den Greifflächen (7) der Zange (6) so zusammengedrückt werden, dass die Materialprobe (10) zwischen den Lagen (11) lose gehalten wird.

14. Verfahren nach einem der Ansprüche 10 bis 13, **dadurch gekennzeichnet, dass** die Materialprobe (10) von der Zange (6) mittig aufgenommen wird.

15. Verfahren nach einem der Ansprüche 10 bis 14, **dadurch gekennzeichnet, dass** die Zange (6) in der Prüfposition so um die Prüfform (2) gebracht wird, dass die Greifflächen (7) konzentrisch um die Prüfform (2) liegen und die Materialprobe (10) zwischen den Lagen (11) in der Prüfform (2) positioniert wird, wobei die Materialprobe (10) durch Anheben der Zange (6) aus der Prüfform (2) gebracht wird, wobei die Lagen (11) lediglich durch Zugbeanspruchung von der Prüfform (2) gelöst werden kann.
